(19)

Europäisches
Patentamt
European
Patent Office
Office européen
des brevets

(11) **EP 2 236 127 A1**

(12) **EUROPEAN PATENT APPLICATION**

(43) Date of publication:
06.10.2010 Bulletin 2010/40

(51) Int Cl.:
*A61K 8/73* *(2006.01)* *A61Q 19/10* *(2006.01)*
*A61K 8/44* *(2006.01)* *A61K 8/46* *(2006.01)*
*A61K 8/60* *(2006.01)* *A61K 8/37* *(2006.01)*
*A61K 8/64* *(2006.01)* *A61K 8/39* *(2006.01)*
*A61K 8/97* *(2006.01)* *A61K 8/365* *(2006.01)*
*A61K 8/98* *(2006.01)* *A61L 15/28* *(2006.01)*
*A61L 15/58* *(2006.01)*

(21) Application number: 10158336.7

(22) Date of filing: 30.03.2010

(84) Designated Contracting States:
**AT BE BG CH CY CZ DE DK EE ES FI FR GB GR
HR HU IE IS IT LI LT LU LV MC MK MT NL NO PL
PT RO SE SI SK SM TR**
Designated Extension States:
**AL BA ME RS**

(30) Priority: 03.04.2009 IT TO20090259

(71) Applicant: Rottapharm S.p.A.
20122 Milano (IT)

(72) Inventors:
• **Tacconi, Elena**
  **I-27036, Mortara (Pavia) (IT)**
• **Trivio, Ramona**
  **I-13100, Prarolo (Vercelli) (IT)**
• **Rovati, Luigi Angelo**
  **I-20052, Monza (Milano) (IT)**

(74) Representative: **Comoglio, Elena et al**
**Jacobacci & Partners S.p.A.**
**Corso Emilia 8**
**10152 Torino (IT)**

(54) **A composition for intimate hygiene**

(57)      A composition for intimate hygiene is described, the which composition comprises one or more active substances selected from the group consisting of dermocompatible detergents, hydrating agents, emollient agents, dermoprotecting agents, dermotrophic agents, lenitive agents, antimicrobial agents, antioxidant agents, deodorising agents, buffering agents, vegetable extracts and combinations thereof, together with xanthan gum as an agent capable of favouring bio-adhesion of the active substances to skin and mucosae. The composition of the invention is designed to be used as a cosmetic, detergent, medicament or medical device, and is prepared in a form suitable for topical application, for instance as a solution, cream or gel, or is impregnated onto a support, such as a cloth or serviette.

EP 2 236 127 A1

**Description**

The present invention relates to a composition for intimate hygiene, particularly a composition for daily intimate hygiene of women susceptible to dryness or easy irritability of the genital area and with poor tolerance to conventional intimate hygiene detergents.

Many women complain about discomfort in their intimate parts, represented at a vulval level by dryness, slight irritation phenomena with itchiness, likely exposure to minor traumas and infections. These phenomena are not true pathological conditions, but annoying discomforts that upset the well being of the subjects affected.

Such discomforts are frequently caused by:

- intimate hygiene done with detergents that are too aggressive for the delicate vulval epithelium, such as soap, alkaline detergents or anyway detergents that are too irritant and also affect the lipid layer that protects the epithelium of the vulval structures;
- genital washes that are too frequent with consequent irritation of the vulval epthelium;
- the use of oral contraceptives, which may result in local hypoestrogenism with hypotrophy of the cutaneous vulvovaginal structures and irritating dryness of the intimate parts;
- the use of very adherent underwear, panty liners, tights, adherent and tight trousers that exert an occlusive action and favour maceration conditions, besides causing mechanical friction micro-traumas;
- underwear made of synthetic or coloured materials, which may result in sensitization and irritation phenomena.

In order to hinder such unpleasant drawbacks, the present invention provides a novel composition for intimate hygiene designed to specifically prevent and/or eliminate the above-mentioned discomforts.

The composition of the invention for the most part contains natural vegetable active substances, which perform a delicate detergent, hydrating, emollient, dermotrophic, dermoprotecting, lenitive, antioxidant, deodorising, and antimicrobial action.

One drawback encountered in the prior art compositions for intimate hygiene is the easiness with which the active substances contained therein are removed from the skin and the mucosae on rinsing.

In order to overcome such a drawback, the composition of the invention comprises one or more active substances selected from the group consisting of dermocompatible detergents, hydrating agents, emollient agents, dermoprotecting agents, dermotrophic agents, lenitive agents, antimicrobial agents, antioxidant agents, deodorising agents, buffering agents, vegetable extracts and combinations thereof, together with a bio-adhesive natural gum, xanthan gum, designed to ensure contact between the active substance(s) and the skin and mucosae, particularly the vulval surfaces and vaginal orifice, for sustained periods of time even after application and rinsing off of the composition.

The matrix of the hygiene composition according to the invention is an aqueous xanthan gum solution. Xanthan gum is a glucose, mannose and glucuronic acid polysaccharide obtained by aerobic fermentation of a substrate made of carbohydrates, nitrogen substances and microelements by the bacterium *Xanthomonas campestris,* which is naturally found on many edible vegetables. By virtue of its high molecular weight (several millions of daltons), xanthan gum in an aqueous solution has pseudo-plastic and viscous colloidal properties. Due to its nature as a hydrophilic colloid, this substance has bio-adhesive properties and guarantees a longer permanence *in situ* of the active substances during and after cleansing. The concentration of xanthan gum in the composition of the invention is preferably within the range from 0.4% to 2.4% by weight over the total weight of the composition, more preferably from 0.6 to 1.6% by weight, even more preferably from 0.6 to 1.0% by weight.

At concentrations higher than 1.6% by weight, xanthan gum forms a gel.

In a preferred embodiment, wherein the composition of the invention is a detergent for intimate hygiene, the composition comprises at least one detergent surfactant. One preferred surfactant is a natural lipoproteic surfactant, such as for example the mixture of sodium cocoyl wheat amino acids known with the name INCI as "Sodium Cocoyl Wheat Amino Acids" (SCWAA).

SCWAA is a dermocompatible natural surfactant capable of restoring the cutaneous protective lipid layer. It is derived from the condensation of coconut oil fatty acids with amino acids obtained from the hydrolysis of wheat proteins. Such a surfactant acts as a detergent without damaging or denaturing the proteins of the horny layer and without causing the side effects of many detergents, such as irritation, dryness, itchiness, and the like. The wheat-borne hydrolyzed proteins are extremely rich in essential amino acids such as arginine, lysine and cysteine, well-known stimulators of the renewal of epidermal keratinocytes and therefore capable of stimulating damaged skin. The condensation of the protein hydrolysate from the proteins of *Triticum vulgare* (wheat) with coconut fatty acids forms an antistatic product highly capable of adhering to skin and to the surface of hairs. Moreover, such a surfactant is able to form a protective film capable of retaining liquids, strengthening the structures of the cutaneous tissue and diminishing the irritating power of other surfactants.

To summarize, SCWAA has the following advantageous features:

- it is an excellent mild, non-irritating detergent suitable for sensitive skins;
- it is easily removed by rinsing;
- it is colourless and odourless;
- it is capable of decreasing the irritating power of the other surfactants optionally-present in the composition.

The preferred concentration of SCWAA in the composition of the invention is within the range from 4% to 20% by weight based on the total weight of the composition.

Further surfactants suitable to be used in the composition of the invention, as an alternative or in combination with the mixture of sodium cocoyl wheat amino acids, are mild dermocompatible surfactant detergents. Among these, we can mention by way of illustration coco-glucoside (INCI designation: Coco-Glucoside) and glyceryl oleate (INCI designation: Glyceryl oleate), which are dermocompatible, foaming, emulsifying detergents with emollient action; disodium laurylether sulfosuccinate (INCI designation: Disodium Laureth Sulfsuccinate) and sodium lauryl sulfoacetate (INCI designation: Sodium Lauryl Sulfoacetate), that is to say mild surfactants that allow for a better hydration of the skin during cleansing; decyl glucoside (INCI designation: Decyl Glucoside), which is a mild hydrating detergent.

As mentioned hereinabove, the composition of the invention comprises one or more active substances, preferably from a natural source.

Among the active substances suitable to be used in the composition of the invention, we can mention by way of illustration:

(i) dermotrophic and dermoprotecting components, among which for instance glyceryl isostearate (INCI designation: Glyceryl Isostearate), glycol distearate (INCI designation: Glycol Distearate), and/or potassium palmitoyl hydrolyzed wheat protein (INCI designation: Potassium Palmitoyl Hydrolyzed Wheat Protein), which are lenitive, emollient, hydrating and elasticizing emulsifying substances, with the object of preserving the physiological elasticity of the skin; Avena sativa extract (INCI designation: Avena Sativa Extract), the hydrolyzed oat protein (INCI designation: Hydrolyzed Oat Protein), and/or the potassium palmitoyl oat protein (INCI designation: Potassium Palmitoyl Oat Protein), which are mild surfactants rich in lipids, carbohydrates and proteins, have lenitive, emollient, hydrating, filmogenic and elasticizing properties, and contribute in maintaining the correct water content and physiological skin elasticity; caprylyl glycol (INCI designation: Caprylyl Glycol), and/or diglycerin (INCI designation: Diglycerin), which have emollient, antimicrobial and wetting properties; maltodextrin, a hydrating and filmogenic skin conditioner;

(ii) vegetable extracts, among which we can mention by way of illustration the *Calendula officinalis* flower hydroglycolic extract and the *Salvia officinalis* hydroglycolic extract. Calendula extract is known for its anti-inflammatory, lenitive, refreshing, re-epithelising and protective action. It acts by a synergistic action of its contents of triterpenic alcohols, flavonoids and polysaccharides and is particularly suitable for the care and restructuring of the epidermis. Salvia extract contains active principles such as salviol, picrosalvin and pinene, which are responsible for the antibacterial, antimycotic, as well as antioxidant activity on free radicals.

Further active substances suitable for use in the composition of the invention are ketoglutaric acid, which is capable of blocking amino radicals (ammonia and urea) and thus performing a deodorising and antioxidant activity; lactic acid, which contributes in maintaining the correct slightly acidic physiologic pH of the vulval surfaces and hinders the development of pathogenic micro-organisms; pasteurised, atomised and dehydrated whey that, together with lactic acid, has a buffering action and assures a sustained acidity on the vulval surfaces; oat milk that, together with diglycerol and maltodextrin, retains a proper hydration and thus the physiological skin elasticity of external genitals.

Optionally, the composition of the invention further comprises fragrances and/or scents selected from those customarily used in detergents for intimate hygiene, which are well tolerated and lack a sensitization capacity.

Preferred concentration ranges for the above-mentioned active substances are the following:

- sodium cocoyl wheat amino acids: from 4% to 16% by weight;
- mixture of disodium laureth sulfsuccinate and sodium lauryl sulfoacetate: from 0.9% to 3.6% by weight;
- mixture of coco-glucoside and glyceryl oleate: from 0.2% to 0.7% by weight;
- decyl glucoside: from 0.2% to 0.7% by weight;
- maltodextrin: from 0.2% to 1.0% by weight;
- mixture of glyceryl isostearate, glycol distearate, potassium palmitoyl hydrolyzed wheat protein: from 0.7% to 3.0% by weight;
- mixture of *Avena Sativa* Extract, hydrolyzed oat protein and potassium palmitoyl oat protein: from 0.14% to 0.6% by weight;
- mixture of caprylyl glycol and diglycerin: from 2% to 8% by weight;
- *Calendula officinalis* flower extract: from 0.25% to 1.0% by weight;
- *Salvia officinalis* extract: from 0.1% to 0.4% by weight;

- ketoglutaric acid: from 0.025% to 0.10% by weight;
- lactic acid: from 0.025% to 0.10% by weight;
- whey: from 0.4% to 1.6% by weight.

The composition of the invention is suitable for use in a detergent product, a cosmetic product, a medicament or a medical device. In this context, the expression "medical device" is used with the meaning defined in the 93/42/CEE Directive related to medical devices.

The composition of the invention is prepared in any form suitable for topic application, particularly in the form of a solution, a cream or a gel. Alternatively, the composition of the invention is used for the manufacture of a hygiene item comprising a support, such as for instance a cloth or serviette, impregnated with the composition preferably in a liquid form. In this context, suitable supports are for example those made of paper, fabric, disposable fabric or similar soft sheet materials.

The following experimental section and examples are provided solely by way of illustration without intending to limit in any way the scope of the invention as defined in the appended claims.

## EXPERIMENTAL SECTION

### Bio-adhesion of the active components to the intimate parts

Bio-adhesion is defined by the ability of a substance to adhere to a biological surface. As to a product for intimate hygiene, the concern is the ability of the hygiene-health substances carried therein to adhere to the cutaneous and mucous surfaces of the external genitals and to remain there even during and after washing.

Xanthan gum is widely used in the food field for conferring suitable viscosity and consistency on liquid or semi-solid food. It also shows interesting bio-adhesive properties on cutaneous and mucous surfaces. It was desired to assess whether it would have favoured bio-adhesion of the active substances contained in the composition of the invention to the cutaneous and mucous surfaces of the external genitals implicated in intimate hygiene actions.

### Materials and methods

An *in vitro* test was used, in compliance with the 76/768 CEE Directive on cosmetic products, which provides for withdrawal from using animals to test cosmetic products, as well as in view of the fact that the realization of a bio-adhesion test in vivo on humans would have encountered considerable technical and organizational difficulties. Therefore, experiments were carried out on the Cutaneous - Mucosal Surface Model (MSCM) devised by Bonferoni et al. 1999 [M.C. Bonferoni, S. Rossi, F. Ferrari and C. Caramella. A modified Franz diffusion cell for simultaneous assessment of drug release and washability of mucoadesive gels. Pharmaceutical Development and Technology, 1999;4:45-53]. The MSCM is set up by soaking a filter paper in a 15% mucin suspension in acetate buffer at pH 4.5 and then drying it in an oven at 60°C. By this treatment, the filter paper takes on physical affinity characteristics and thus the ability of adhering substances that contact it, very similar to the vulval skin.

The apparatus and method described by the above-mentioned authors was used to measure the ability of adhering the test substances. The apparatus consists of a cylindrical chamber 20 mm in diameter and 5 mm in height (1.6 ml - 1.3 ml volume if the base is coated with biadhesive and filter paper) provided with two opposed lateral arms to allow for inlet (top arm) and outlet (bottom arm) of the liquid used for washing and removing the substance the bio-adhesion of which is to be assayed. A circle 20 mm in diameter cut out from the filter paper subjected to the above-described treatment is adhered onto the bottom of the chamber by way of a biadhesive. In order to recreate the thermodynamic conditions of the vulval surface, the chamber and washing liquid are thermostated at 30°C.

The following preparations were investigated:

Control 1: De-carbonated distilled water containing 0.2% fluorescein isothiocyanate (TRACER) used as a probe to measure the bio-adhesion ratio.

Control 2: De-carbonated distilled water containing 0.2% fluorescein isothiocyanate (TRACER) and further containing 0.4% xanthan gum.

Control 3: De-carbonated distilled water containing 0.2% fluorescein isothiocyanate (TRACER) further containing 0.8% xanthan gum.

Composition of the invention (cf. example 1), to which 0.2% fluorescein isothiocyanate (TRACER) was added.

After the mucined filter paper circle was placed onto the bottom of the chamber, a 100 mg aliquot of test liquid is layered on top of it and left there for 30 minutes. Subsequently, the cell is filled with the washing liquid (de-carbonated water) and perfusion of the cell with de-carbonated water kept at 30°C is started. The perfusion is carried out by an HPLC pump at a constant flow rate of 0.3 ml/minute. The washing liquid flow through is collected from the chamber into a graduated glass and kept under stirring with a magnetic stirrer. After 5 minutes of perfusion, the collected volume is measured, 0.2 ml of liquid are collected, the fluorescein concentration is measured with a fluorimeter and the amount of eluted (washed off) fluorescein is calculated, expressing it as the percentage of the total fluorescein thiocyanate fed into the chamber (0.2 mg). This operation is repeated at 10, 20, 30, 45, 60, 120 and 180 minutes. After each measurement, the collected liquid is put back into the glass.

### Results

4 assessments were made for each of the test preparations. Based upon the TRACER eluted, the percentage of TRACER still adhering to the mucined filter paper was calculated at the different time points (TA%) and correlated to the elution times. A good linear correlation between log(TA%) and elution time was observed.

The constants of the following equation were calculated by the least square method:

$$\log(TA\%) = k \times \min + C \text{ (equation 1)}$$

wherein k represents the elution constant and C the intercept on the TA% axis (on a logarithmic scale). In general, a good linear correlation was found between the figures subsequent to the 5-minute time point (time required to reach the stationary state) and TA% > 10.

The k values obtained with the test preparations along with the relative standard errors are shown in the table 1 below.

Table 1

| k values with relative standard errors. $t_{1/2}$ with limits at 95% | | | |
|---|---|---|---|
| Preparation | k (SE) | $R^2$ | $t_{1/2}$ min (limits 95%) |
| Control 1 | -0.074 (0.007) | 0.9966 | 4.07 (3.72-4.49) |
| Control 2 | -0.0031 (0.0005) | 0.9769 | 97 (84-116) |
| Control 3 | -0.0021 (0.0003) | 0.9646 | 143 (125-165) |
| Composition | -0.0017 (0.0007) | 0.9711 | 177 (151-215) |

The results show an excellent linear correlation between log(TA%) and time, as evidenced by $R^2$ values (correlation coefficient[2]) that approximate 1.00.

The following results were obtained with the test preparations.

Control 1 (water with TRACER)

The TRACER dissolved in water elutes rapidly from MSCM, with an elution $t_{1/2}$ of 4.07 minutes only.

Control 2 (solution with 0.4% xanthan gum and TRACER)

The TRACER elutes slowly (elution $t_{1/2}$ = 97 minutes). Thus, 0.4% xanthan gum increases 24-fold the retention of the TRACER on MSCM.

This is an unexpected and surprising result. In fact, while the bio-adhesive properties of xanthan gum are well known, not so was its ability to promote bio-adhesion of substances carried therein onto MSCM.

Control 3 (solution with 0.8% xanthan gum and TRACER)

Very surprising is the increase in TRACER bio-adhesion promoted by 0.8% xanthan gum, with an elution $t_{1/2}$ of 143 minutes, significantly higher by 51 % than the $t_{1/2}$ of 97 minutes obtained with 0.4% xanthan gum and 35 times higher than the elution $t_{1/2}$ of the TRACER dissolved in water.

Composition of the invention (cf. example 1) with the TRACER

Even more surprisingly, the elution of the TRACER from MSCM is further slowed down by the Composition. In fact, the elution $t_{1/2}$ is of 177 minutes, that is 24% higher than the elution time of the TRACER from the aqueous 0.8% xanthan gum solution. It is thus evident and surprising that the active substances present in the composition slow down still further the elution of the TRACER.

***Conclusion***

Surprisingly, xanthan gum promotes bio-adhesion of the TRACER (0.2% fluorescein isothiocyanate) to MSCM, either in aqueous solution or mixed with other active substances, as in the composition.
Since the bio-adhesion of the TRACER can be considered as an indicator of the quantitative bio-adhesion parameters of the other components of the composition of the invention, it can be concluded that xanthan gum favours the bio-adhesion thereof to the vulval surfaces and prolongs the activity and effectiveness thereof.

***Epicutaneous test to assess the irritating potential (Patch Test)***

***Method***

20 $\mu$l of composition of the invention (cf. example 1) diluted 1:10 in water were applied to the skin of the back of 30 healthy adult volunteers, with a perfectly intact skin, by a Finn chamber. The Finn chamber ensures an occlusive application and consists of a small aluminium cell 7 mm in diameter, containing a blotting paper disk soaked in the test sample. It is fixed to the skin of the subjects through a patch already tested for its harmlessness to skin. The skin reactions are assessed 15 minutes, 1 hour and 24 hours after the application and are scored in the following way.

Erythema

| Intensity | Score |
|---|---|
| Absent | 0 |
| Mild (hardly visible) | 1 |
| Well visible | 2 |
| Moderate | 3 |
| Serious (beet red with possible formation of light eschars) | 4 |

Edema

| Intensity | Score |
|---|---|
| Absent | 0 |
| Very mild (hardly visible) | 1 |
| Light | 2 |
| Moderate (about 1 mm raised skin) | 3 |
| Strong (extended swelling beyond the application area) | 4 |

Based on the average score obtained in the subjects who underwent the test, the test product is scored in the following way:

Classification of tolerability in the Patch test

| Average score | Classification |
|---|---|
| 0.0 - 0.5 | Non-irritating (Good tolerability) |
| 0.5 - 2.0 | Slightly irritating |
| 2.0 - 5.0 | Moderately irritating |
| >5.0 | Highly irritating |

*Results*

Table 2 shows the average scores and the relative standard errors collected from 30 healthy volunteers.

Table 2. Erythema and edema scores after application of the composition.

| Averages and (SE) from 30 subjects | | | | | |
|---|---|---|---|---|---|
| Subjects (30) | Erythema 15' | Edema 15' | Erythema 1 h | Edema 1 h | Erythema 24 h | Edema 24 h |
| 3M;27F | 0.20 (0.07) | 0.00 (0.00) | 0.17 (0.07) | 0.00 (0.00) | 0.00 (0.00) | 0.00 (0.00) |

Average and general SE (180 assessments): 0.055 (0.0041).

*Conclusion*

The skin tolerability of the composition of the invention assayed by Patch Test on 30 healthy volunteers (3 males and 27 females) was good (much lower than the irritating threshold).

*Pro-sensitization test*

An *in vitro* test was used, in compliance with the 76/768 CEE Directive, which for cosmetic products provides for withdrawal from using animals to test cosmetic products. On the other hand, it is not ethically acceptable to perform a sensitization test for a cosmetic on human subjects.

The *in vitro* test used estimates the presence or absence of pro-sensitization effects by products designed to be used on skin, by using a cellular model to assess the immunological reactivity of typical immune cells (monocytes) exposed to extended contact (48 hours) with the test products.

The test was carried out on the monocytic cell line THP-1 as this cell type is greatly implicated in the immune responses of the skin. These cells were assessed for the modulation of the expression of two co-stimulatory molecules, CD80 (B7.1) and CD86 (B7.2), by using, as positive controls, typical contact sensitization substances, that is nickel sulphate (Ni-SO$_4$•6H$_2$O) and Kathon CG. Nickel is capable of evoking immune reactions *in vivo* of the allergenic type and has been largely used *in vitro* to study the modulation of the immune response. Kathon CG is a preservative and is graded from the toxicological point of view as a primary irritant and potentially sensitizing agent.

The recognition of the antigen by the TCR (T Cell Receptor) of T lymphocytes (signal 1) is not functional for the maturation of an effective immune response if it does not occur at the membrane of a cell presenting the same antigen and capable of giving a further signal (signal 2 or co-stimulus) qualitatively essential for the definition of the response type (humoral, cell-mediated, etc.). Both B7.1 and B7.2 (collectively referred to as B7) are membrane glycoproteins present on the surface of many antigen-presenting cells (dendritic cells, Langerhans cells, monocytes/macrophages, different cell lines among which keratinocytes) and they act as co-stimuli. In fact, both the molecules are ligands for a glycoprotein called CD28, present on T lymphocyte membranes. Priming of the CD28/B7 ligand/receptor system prevents apoptosis (programmed cell death) of T cells and cooperates in sustaining the proliferation and differentiation thereof.

In the first "physiological" phases of the immune response, B7.2 is constitutively expressed and modulates both the Th1 and Th2 responses. As the immune response progresses, B7.1 also is up-regulated and increases the co-stimulatory signal intensity, with expansion of T cells and production of several cytokines. Moreover, B7.1 is preferentially up-regulated during the acute phase of autoimmune responses.

The increased expression of these co-stimulatory molecules on monocytes is thus indicative of activation of an immune response consequent to exposure to a potentially allergenic antigen. In fact, functionally, the expression of co-stimulatory molecules on this cell type corresponds to acquisition of competence for presenting the antigen in the typical site (skin in our case) where contact sensitization occurs *in vivo.*

*Method*

The sample (composition prepared as in Example 1) was dissolved in ethanol, directly diluted at different concentrations in the cell culture medium and subjected to a preliminary cytotoxicity test on THP-1 cells in order to estimate the concentration at which the substance could be used *in vitro* without causing cell death, the which induces alterations in the results. The cell growth medium left under the same experimental conditions was used as the negative control.

Execution of the preliminary MTT test

The key reagent is 3-[4,5-dimethylthiazol-2-yl]-2,5-diphenyl tetrazolium bromide or MTT, a substance that gives a yellow colour in aqueous solution. The mitochondrial dehydrogenase of viable cells cleaves the tetrazolium ring, forming water-insoluble violet purple-coloured formazan crystals. The crystals are dissolved in acidified isopropanol and the resulting violet solution is measured by a spectrophotometer. An increase or decrease in viable cells results in a concomitant change in the amount of formazan formed, which may be considered as an indicator of the cytotoxicity level caused by exposure to the tested substances. After treatment, the cells are washed with PBS and the MTT solution is added to each well, with subsequent incubation at 37°C. At the end of the incubation period, the medium-MTT is removed and the MTT-solubilising solution is added to each well. The plate is stirred on a plate stirrer for 20-30 minutes, making sure that all the crystals are dissolved and have formed a homogeneous solution. The result is expressed as:

$$\% \text{ cell survival} = 100 \text{ x (OD treated cells)/(OD non-treated cells)}$$

### Execution of the sensitization test

After exposure, the cells were examined for viability by Tripan Blue staining and microscopic observation in a hemocytometer. The cells were collected, washed in an isotonic buffer (PBS) and labelled with a fluorescein-antibody directed towards B7.1 or B7.2. After further washes to remove the antibody excess, the cells were placed into a flow cytofluorimeter to assess the MFI (Mean Fluorescence Intensity), which is proportionate to the number of labelled molecules per cell, and therefore representative of the level of expression of the investigated co-stimulatory molecules.
Also, morphological qualitative parameters (altered cell volume, modified cell granulation, etc.) linked to cell necrosis and apoptosis were detected, the which events are closely related to the allergic elicitation process.
The positive controls are represented by THP-1 cultures exposed to nickel sulphate and Kathon CG. The concentrations of the two pro-sensitizers used as the reference were selected among those that resulted "non-cytotoxic" in the preliminary MTT test. The MFI of the untreated THP-1 cells and the MFI of the THP-1 cells reacted with a non-specific fluoresceinated monoclonal antibody were assessed to be used as the negative control (baseline fluorescence).

### Results

The results of the analysis of the THP-1 monocyte line for expression of co-stimulatory molecules by flow cytofluorimeter after 48 hours of reaction with the two tested concentrations of the sample and the controls, corrected for the negative control, are shown in Table 3.

Table 3. Expression of pro-sensitizing molecules

| Samples | CD80 (MFI*) | CD86 (MFI*) |
|---|---|---|
| 20 $\mu$g/ml nickel sulphate | 24.08 | 61.92 |
| 10 $\mu$g/ml nickel sulphate | 16.45 | 28.75 |
| 4 $\mu$g/ml nickel sulphate | 3.22 | 3.69 |
| 15 $\mu$g/ml Kathon CG | 18.7 | 18.88 |
| 3 $\mu$g/ml Kathon CG | 2.93 | 4.99 |
| 0.2 $\mu$l/ml Composition | -0.02 | -0.13 |
| 0.04 $\mu$l/ml Composition | -0.06 | 0.13 |
| *MFI = Mean Fluorescence Intensity: geometric mean of the fluorescence intensity from the cells stained with the fluoresceinated antibody, which is proportionate to the number of stained molecules per cell. | | |

By observing the behaviour of the THP-1 culture in contact with nickel, a typical allergenic substance, it can be seen that this triggers:

- a high increase in both the labels;

- a direct correlation of the increase in response intensity and the concentration;
- a detectable effect even at very low doses (4 μg/ml).

The low tested dose of nickel sulphate (4 μg/ml) corresponds to approximately 1 ppm of nickel, a value that is about that of the allergenic threshold dose in subjects already sensitized and on irritated skin. In this experiment, such a concentration was established to be able to cause a detectable increase in CD80, compared to untreated controls (data not shown).

However, the concentration capable of causing an allergic reaction in most of the sensitive subjects is at much higher values, over 100 ppm of nickel in contact with intact and healthy skin.

Culture of THP-1 in contact with Kathon CG showed:

- an increase in both the labels;
- a direct correlation of the concentration and the increase in response intensity;
- a detectable effect even at very low concentrations (3 μg/ml).

In summary, at the tested concentrations, the composition of the invention did not show a modulation of the labels under investigation, did not show cytotoxic effects on the cell culture used, did not induce apoptotic effects on the cells under investigation, did not increase the expression of the two tested labels in monocytes *in vitro* even at concentrations of 0.2 μg/ml, thereby showing that it does not exhibit a potential monocyte/macrophage-mediated stimulus of the immune system.

It can therefore be concluded that the composition does not show detectable pro-sensitization activities in the pro-sensitization test.

## Examples

The hereinbelow reported preparations illustrate embodiments of the present invention and as such must not be regarded in any way as limiting the invention itself.

Particularly, the possibility is mentioned of using alternative equipment, amounts and excipients compared to those explicitly mentioned in the preparation examples illustrated hereinbelow, depending on the formulation and technology requirements. The selection of the suitable equipment, excipients and amounts is well within the skills of the average technician of the cosmetic preparation and pharmaceutical arts and does non require any inventive activity.

Further, it should be intended that the dosage of the active substances specified in the examples is purely illustrative and may be varied as the specific occasion requires.

The inclusion into the composition of the invention of further complementary substances designed to contribute to a further improvement in the health and cosmetic effects of the mentioned active substances is within the scope of the present invention. The average skilled person of the art is able to identify on a case-by-case basis the required formulation modifications, by taking into account several factors such as quality, quantity and rheological characteristics of the complementary substances.

Example 1. Preparation of a 4 kg batch of the composition according to the invention

1. Preparing the aqueous phase

Disperse in a turboemulsifier with stirring blades into 3098 g of water at 50°C:

| | |
|---|---|
| 2.00 g* | ketoglutaric acid |
| 32.00 g* | lactic acid |
| 36.00 g* | pasteurised, atomised and dehydrated whey |
| 20.00 g* | *Calendula officinalis* flower hydroglycolic extract |
| 8.00 g* | *Salvia officinalis* hydroglycolic extract |
| up to complete dissolution. | |

2. Add:

32.00 g*    Xanthan gum

3. Adding of the dermotrophic detergent surfactants

With blade-stirring, in vacuo, at 30°C

26.80 g*    mixture of Coco-glucoside and Glyceryl Oleate
14.80 g*    Decyl Glucoside
72.00 g*    mixture of disodium laurylether sulfosuccinate and sodium lauryl sul foacetate
320.00 g*    30% Sodium cocoyl wheat amino acids

4. Adding of the dermotrophic and dermoprotective components

With blade-stirring, in vacuo, and at 30°C, add:

20.00 g* Maltodextrin
58.40 g* mixture of Glyceryl Isostearate, Glycol Distearate, Potassium Palmitoyl hydrolyzed wheat protein (1.5:1.5:1)
11.20 g* mixture of *Avena Sativa* extract, hydrolyzed oat protein, potassium palmitoyl oat protein (4:1:1)
160.00 g* mixture of Caprylyl Glycol and Diglycerin (1:7)

5. Adding of 88.00 g of fragrances

6. Unload from the turboemulsifier into suitable containers.

The sequence and type of operations reflect the conditions used to prepare the composition described in Example 1. Modifications thereof are feasible based on the available equipment, batch size, and productive custom.

The dosages marked with * are the ones used in the example and are to be considered purely indicative both as regards the preparation of 4 kg batches and the ratios to be used in different size batches.

## Claims

1.  A composition for intimate hygiene, comprising one or more active substances selected from the group consisting of dermocompatible detergents, hydrating agents, emollient agents, dermoprotecting agents, dermotrophic agents, lenitive agents, antimicrobial agents, antioxidant agents, deodorising agents, buffering agents, vegetable extracts and combinations thereof, together with xanthan gum as an agent capable of favouring bio-adhesion of the active substances to skin and mucosae.

2.  The composition according to claim 1, comprising xanthan gum at a concentration from 0.4% to 2.4% by weight over the total weight of the composition.

3.  The composition according to claim 1 or 2, comprising at least one natural lipoproteic surfactant.

4.  The composition according to any of claims 1 to 3, wherein the at least one natural lipoproteic surfactant is a mixture of sodium cocoyl wheat amino acids.

5.  The composition according to claim 4, comprising the mixture of sodium cocoyl wheat amino acids at a concentration of 4% to 20% by weight over the total weight of the composition.

6.  The composition according to any of claims 1 to 5, comprising at least a further dermocompatible detergent selected from the group consisting of Disodium Laureth Sulfsuccinate, Sodium Lauryl Sulfoacetate, Coco-Glucoside, Glyceryl oleate, Decyl Glucoside and combinations thereof.

7.  The composition according to any of claims 1 to 6, comprising at least a dermotrophic agent selected from the group consisting of maltodextrin, Glyceryl Isostearate, Glycol Distearate, Potassium Palmitoyl Hydrolyzed Wheat Protein, Avena Sativa Extract, Hydrolyzed Oat Protein, Potassium Palmitoyl Oat Protein, Caprylyl Glycol, Diglycerin and combinations thereof.

8. The composition according to any of claims 1 to 7, comprising *Calendula officinalis* flower extract and/or *Salvia officinalis* extract.

9. The composition according to any of claims 1 to 8, comprising ketoglutaric acid as an antioxidant and deodorising agent.

10. The composition according to any of claims 1 to 9, comprising lactic acid in combination with milk serum as buffering agents.

11. The composition according to any of claims 1 to 10, as a detergent, cosmetic, medicament or medical device.

12. The composition according to any of claims 1 to 11, in a form suitable for topical application, preferably in the form of a solution, a cream or a gel.

13. A hygiene product, comprising a support, such as a cloth or serviette, impregnated with a composition according to any of claims 1 to 12.

Europäisches
Patentamt

European
Patent Office

Office européen
des brevets

# EUROPEAN SEARCH REPORT

Application Number

EP 10 15 8336

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 03/043599 A1 (BEIERSDORF AG [DE]; ALBRECHT HARALD [DE]; KOCH PETRA [DE]; KOHUT MICHA) 30 May 2003 (2003-05-30) * page 12 * | 1,6,11, 12 | INV. A61K8/73 A61Q19/10 A61K8/44 A61K8/46 |
| X | GB 2 182 339 A (AVENT MEDICAL LTD AVENT MEDICAL LTD [HK]) 13 May 1987 (1987-05-13) * example 2 * | 1,2,11, 12 | A61K8/60 A61K8/37 A61K8/64 A61K8/39 |
| X | US 6 506 375 B1 (BARR TERESA LEIGH [US]) 14 January 2003 (2003-01-14) * line 1 - column 9, line 20 * | 1,2,7, 11,12 | A61K8/97 A61K8/365 A61K8/98 A61L15/28 |
| X | DE 20 2008 003980 U1 (KNEIPP WERKE KNEIPP MITTEL ZEN [DE]) 24 July 2008 (2008-07-24) * example 1 * | 1,8,11, 12 | A61L15/58 |
| X | EP 1 661 552 A1 (FO S P A SA [IT]) 31 May 2006 (2006-05-31) * example 2 * | 1 | |
| X | WO 90/14110 A1 (VILAIN JEAN [GB]) 29 November 1990 (1990-11-29) * claim 1 3 9 12 13 * | 1,11-13 | **TECHNICAL FIELDS SEARCHED (IPC)** A61K A61Q A61L |
| X | WO 03/032948 A2 (MCNEIL PPC INC [US]) 24 April 2003 (2003-04-24) * claims 1, 11-15, 31, 41-45, 65, 75-79 * * example 5 * | 1-13 | |
| A | EP 1 842 536 A1 (C Y L PHARMAZEUTIKA GMBH [AT]) 10 October 2007 (2007-10-10) * claim 1 * | 1-13 | |

The present search report has been drawn up for all claims

| Place of search | Date of completion of the search | Examiner |
|---|---|---|
| The Hague | 16 April 2010 | Sierra Gonzalez, M |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 10 15 8336

This annex lists the patent family members relating to the patent documents cited in the above-mentioned European search report.
The members are as contained in the European Patent Office EDP file on
The European Patent Office is in no way liable for these particulars which are merely given for the purpose of information.

16-04-2010

| Patent document cited in search report | | Publication date | Patent family member(s) | | Publication date |
|---|---|---|---|---|---|
| WO 03043599 | A1 | 30-05-2003 | DE 10157541 A1 | | 12-06-2003 |
| | | | EP 1453480 A1 | | 08-09-2004 |
| | | | JP 2005513022 T | | 12-05-2005 |
| | | | US 2004266645 A1 | | 30-12-2004 |
| GB 2182339 | A | 13-05-1987 | NONE | | |
| US 6506375 | B1 | 14-01-2003 | NONE | | |
| DE 202008003980 | U1 | 24-07-2008 | NONE | | |
| EP 1661552 | A1 | 31-05-2006 | NONE | | |
| WO 9014110 | A1 | 29-11-1990 | AU 5561690 A | | 18-12-1990 |
| | | | EP 0472575 A1 | | 04-03-1992 |
| | | | JP 4505267 T | | 17-09-1992 |
| WO 03032948 | A2 | 24-04-2003 | AU 2002335001 A1 | | 28-04-2003 |
| | | | CA 2463711 A1 | | 24-04-2003 |
| | | | CN 1630509 A | | 22-06-2005 |
| | | | EP 1435907 A2 | | 14-07-2004 |
| | | | JP 2005511530 T | | 28-04-2005 |
| | | | KR 20040063909 A | | 14-07-2004 |
| | | | MX PA04003656 A | | 20-06-2005 |
| | | | US 2003091540 A1 | | 15-05-2003 |
| EP 1842536 | A1 | 10-10-2007 | AT 503385 A1 | | 15-10-2007 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **M.C. Bonferoni ; S. Rossi ; F. Ferrari ; C. Caramella.** A modified Franz diffusion cell for simultaneous assessment of drug release and washability of mucoadesive gels. *Pharmaceutical Development and Technology,* 1999, vol. 4, 45-53